# EUROPÄISCHE PATENTSCHRIFT

(11) **EP 1 897 580 B1**
(45) Veröffentlichungstag und Bekanntmachung des Hinweises auf die Patenterteilung: **06.07.2016**
(21) Anmeldenummer: 07014723.6
(22) Anmeldetag: 26.07.2007
(51) Int. Cl.: A61M 25/00

(54) **Katheter-Set**
Catheter set
Ensemble de cathéter

(30) Priorität: 06.09.2006 DE 202006013663 U; 18.04.2007 DE 102007018275
(43) Veröffentlichungstag der Anmeldung: 12.03.2008
(73) Patentinhaber: Medical Service GmbH, 75378 Bad Liebenzell (DE)
(72) Erfinder: Burkert, Ralf, 75378 Bad Liebenzell (DE); Bobzien, Sonja, 71263 Weil der Stadt (DE)
(74) Vertreter: Grünecker Patent- und Rechtsanwälte PartG mbB

(56) Entgegenhaltungen:
- EP-A- 0 913 164
- WO-A-2004/060441
- DE-U1- 20 021 431
- DE-U1-202005 009 946
- US-A- 5 593 394
- US-A1- 2002 156 440
- US-A1- 2003 060 807

## Beschreibung

Die Erfindung betrifft ein Katheter-Set, umfassend eine aus einem folienartigen Material hergestellte Verpackung und einen Blasenkatheter mit einem flexiblen Katheterschaft und einer Katheterspitze, wobei die Verpackung einen im Wesentlichen röhrenförmigen Katheteraufnahmeraum aufweist, in den der Blasenkatheter eingelegt ist, und wobei an dem der Katheterspitze zugeordneten Ende des Katheteraufnahmeraums eine Austrittsstelle für den Blasenkatheter vorgesehen ist.

Die gattungsbildende US 2003/060807 A1 umfasst eine Katheteranordnung mit einer Verpackung, wobei die Verpackung als Auffangbehältnis dient. Am einführbaren Ende des Katheters weist die Verpackung eine wiederverschließbare Öffnung auf, um bis zur Entsorgung des Katheters eine hygienische Aufbewahrung zu ermöglichen. Um die Entnahme zu erleichtern, sind an der Entnahmeöffnung des Katheters Griffbereiche angeordnet. Zwischen den Griffbereichen ist ein zusammenschiebbarer Abschnitt vorgesehen, der die Entnahme des Katheters ermöglicht, ohne diesen berühren zu müssen. Der zwischen den beiden Griffbereichen zusammenstauchbare, bzw. zusammenschiebbare Bereich ist rippenartig geformt und vereinfacht die Entnahme des Katheters. Nach Öffnung der Verpackung kann durch ein Zusammenschieben des stauchbaren Bereichs der Verpackung der Kopf des Katheters freigelegt werden. An den Griffbereichen, die sich jeweils zu beiden Enden des stauchbaren Bereichs befinden, kann die Katheterverpackung nun gegriffen und der Katheter eingeführt werden.

Ein weiteres Katheter-Set, wie es beispielsweise aus der DE 20 2005 009 946 U1 bekannt geworden ist, erlaubt eine sterile Verpackung eines Blasenkatheters, der zum Gebrauch durch die Austrittsstelle aus der Verpackung geführt wird. Der Blasenkatheter tritt mit der Katheterspitze voran 5 aus der Verpackung und wird direkt in die Harnröhre zur Harnblase eines Patienten geführt. Durch die gebrauchsfertige Lage des Blasenkatheters innerhalb des Katheteraufnahmeraums der Primär-Verpackung und durch die Flexibilität des Verpackungsmaterials wird ein unmittelbarer Kontakt zwischen dem Blasenkatheter und den ihn haltenden bzw. führenden Händen vermieden. Um mittels des Katheters abgeführten Urin aufzufangen, weist das Katheter-Set einen an den Katheteraufnahmeraum anschließenden Urinaufnahmeraum auf. Der Urin wird durch den Katheteraufnahmeraum zum Urinaufnahmeraum geleitet. Der Katheteraufnahmeraum kann durch eine Rückflusssperre vom Urinaufnahmeraum getrennt werden, so dass im Urinaufnahmeraum aufgefangener Urin darin verbleibt und nicht auslaufen kann.

Bei bekannten Katheter-Sets kann ein Teil des Urins im Katheteraufnahmeraum verbleiben, da dieser z.B. in Kanten des Katheteraufnahmeraums anhaften kann oder einfach nicht vollständig in den Urinaufnahmeraum abfließt. Dieser Teil des Urins kann nach Abschluss des Gebrauchs des Katheters durch die Austrittsstelle aus der Verpackung heraustreten, da die Austrittsstelle durch Aufreißen einer Sollbruchstelle der Verpackung geöffnet ist. Letzteres erschwert die Handhabung des Katheter-Sets nach dessen Verwendung und beeinträchtigt die Hygiene des Katheterisierungsvorgangs.

Der Erfindung liegt die Aufgabe zugrunde, ein Katheter-Set bereitzustellen, welches die Nachteile des Standes der Technik vermeidet, wobei die Verpackung verbessert und insbesondere die Handhabbarkeit erleichtert werden soll.

Diese Aufgabe wird durch das Katheter-Set des unabhängigen Anspruchs gelöst. Die abhängigen Ansprüche stellen bevorzugte Ausführungsformen der Erfindung dar.

Erfindungsgemäß ist der Katheteraufnahmeraum Teil eines Urinaufnahmeraums. Weiter ist an der Austrittsstelle ein wiederverschließbares Verschlusselement vorgesehen. D.h. die Austrittsstelle ist durch das Verschlusselement wiederverschließbar.

Bei dem erfindungsgemäßen Katheter-Set dient die Verpackung für den Blasenkatheter zugleich als Sammel- bzw. Aufnahmebeutel für den durch den Blasenkatheter abgeleiteten Urin. An dem der Katheterspitze gegenüberliegenden Ende des Blasenkatheters ist zweckmäßigerweise ein Blockierelement vorgesehen, welches ein vollständiges Herausziehen des Blasenkatheters aus der Verpackung verhindert und während des Katheterisierungsvorgangs den Katheteraufnahmeraum bzw. die Austrittsstelle nach außen abdichten kann. Dazu ist das Blockierelement in seiner äußeren Abmessung an die Abmessung der Austrittsstelle derart angepasst, dass diese mit dem Blockierelement als eine Art Stöpsel verschließbar ist, so dass während des Katheterisierungsvorgangs kein Urin ungewollt seitlich neben dem Katheter aus dem Katheteraufnahmeraum fließen kann.
Beim typischen Einsatz des erfindungsgemäßen Katheter-Sets wird der Blasenkatheter aus der Verpackung geschoben und in die Harnblase des Patienten eingeführt. Der Urin fließt während des Katheterisierungsvorgangs direkt in den Urinaufnahmeraum, mit anderen Worten in die Verpackung, und der Blasenkatheter wird anschließend aus dem Körperhohlorgan zurückgezogen und in die Verpackung geschoben. Abschließend wird die Austrittsstelle mittels des Verschlusselements wieder verschlossen. Durch das Verschlusselement kann die Verpackung nach Rückführung des Blasenkatheters wieder dicht verschlossen werden und die Verpackung samt des verwendeten Blasenkatheters und des in ihr gespeicherten Urins entsorgt werden. Da das Verschlusselement an der Austrittsstelle angeordnet ist, kann nach dem Katheterisierungsvorgang kein Urin aus dem Katheteraufnahmeraum austreten. Weiter kann das erfindungsgemäße Katheter-Set hinsichtlich seiner Gesamtabmessung relativ klein ausgeführt werden, da der Katheteraufnahmeraum teilweise oder komplett zugleich als Teil des Urinaufnahmeraums genutzt wird.

Bevorzugt weist der Katheteraufnahmeraum eine an die Austrittsstelle angrenzende bzw. diese ausbildende Führungshülse auf. Beim Ausführen durchtritt der Blasenkatheter die Führungshülse und wird durch diese in seiner Austrittsrichtung stabilisiert. Die Führungshülse dient aufgrund dieser Richtungsstabilisierung als Einführhilfe beim Einführen des Blasenkatheters in ein Körperhohlorgan. Das Verschlusselement kann dabei als Verschlussklappe an der Führungshülse ausgebildet sein. Eine derartige Verschlussklappe lässt sich einfach durch Aufklappen öffnen und durch Zuklappen wieder verschließen. Das Verschlusselement kann dabei nicht verlorengehen, da die Klappe beweglich am Verschlusselement befestigt ist.

Dabei sind die Verschlussklappe und die Führungshülse sehr vorteilhaft einstückig aus Kunststoff hergestellt, wobei die Verschlussklappe und die Führungshülse über mindestens einen flexiblen Kunststoffstreifen miteinander verbunden sind. Diese Ausführungsform der Verschlussklappe mit der Führungshülse ist sehr einfach und kostengünstig herstellbar.

Vorteilhaft weist die Führungshülse an ihrer Innenoberfläche in Katheteraustrittsrichtung verlaufende Führungsrippen auf, so dass die in der Regel mit einem kleineren Durchmesser als der Innendurchmesser der Führungshülse ausgeführte Katheterspitze noch besser geführt wird. Insbesondere wenn eine Aktivierung der Katheteroberfläche mittels eines Gels als Gleitmittel vor dessen Applikation vorgesehen ist, wird durch die Führungsrippen eine gleichmäßige Verteilung des Gleitmittels auf der Katheteroberfläche gewährleistet. Insbesondere wird ein Abstreifen des Gleitmittels von Teilen der Katheteroberfläche durch die Führungshülse vermieden.

Zum besseren Abdichten beim Verschließen der Führungshülse mittels des Verschlusselements können die Führungshülse und/oder das Verschlusselement eine umlaufende Dichtlippe aufweisen.

Bei einem erfindungsgemäßen Katheter-Set können in zumindest einem Längenabschnitt des Katheteraufnahmeraums Führungselemente an der Außenoberfläche der Verpackung vorgesehen sein, wobei die Führungselemente in Längsrichtung des Katheteraufnahmeraums der Verpackung dem Verlauf des Katheteraufnahmeraums zumindest teilweise folgen.
Die Führungselemente sind entlang der länglichen Ausdehnung des Katheteraufnahmeraums, mit anderen Worten entlang der axialen Erstreckung des Katheterschafts, angeordnet. Zum Aus- bzw. Einführen des Blasenkatheters aus der Verpackung greift ein Benutzer mit beiden Händen außen an dem Katheteraufnahmeraum an, fixiert mit einer Hand die Verpackung am Katheterschaft und schiebt mit der anderen Hand den Blasenkatheter aus der Verpackung. Durch die Führungselemente wird die Griffigkeit der üblicherweise glatten Außenoberfläche der Verpackung erhöht und ein Ab- bzw. Verrutschen der den Blasenkatheter greifenden Finger vermieden. Auf diese Weise wird bei dem erfindungsgemäßen Katheter-Set bzw. Katheter-System ein vereinfachtes und erleichtertes Führen des flexiblen Katheterschafts sichergestellt. Durch die verbesserte Handhabbarkeit des Blasenkatheters ist die Hygiene beim Gebrauch des erfindungsgemäßen Katheter-Sets im Vergleich zu herkömmlichen Katheter-Sets erhöht, und das Ein- und/oder Ausführen eines Katheters aus der Verpackung wird erheblich erleichtert.

Das Material der Verpackung ist in der Regel ein Kunststoff; es ist jedoch auch denkbar, ein Leichtmetall oder ein beschichtetes Gewebe oder Kombinationen davon einzusetzen. Durch Verkleben, Verschweißen und/oder Vernähen des folienartigen Materials erhält die Verpackung eine flächige Gestalt. Um eine sterile Lagerung des Blasenkatheters sicherzustellen, ist der Katheteraufnahmeraum an der Austrittsstelle verschlossen. Vor, bzw. an der Austrittsstelle kann eine Sollbruchnaht vorgesehen sein, die vor oder beim Ausführen des Blasenkatheters durchbrochen wird. Ein vor dem Austritt des Blasenkatheters zu öffnendes Verschlusselement, z.B. eine Kappe oder dergleichen, ist dann vom Material der Verpackung, z.B. Kunststofffolie, mit umschlossen.

In einer bevorzugten Ausführungsform des erfindungsgemäßen Katheter-Sets sind die Führungselemente im Bereich der Austrittsstelle vorgesehen. Beim Aus- bzw. Einführen des Blasenkatheters greift ein Benutzer die Verpackung typischerweise nahe der Austrittsstelle an, um den Blasenkatheter in kontrollierter Weise in den Körper des Patienten einzuführen, so dass sich die Führungselemente in diesem Bereich als besonders vorteilhaft erweisen.

In einer weiteren bevorzugten Ausführungsform des erfindungsgemäßen Katheter-Sets erstrecken sich die Führungselemente über die gesamte Länge des Katheteraufnahmeraums. In dieser Ausführungsform sind die Führungselemente entlang des gesamten Katheteraufnahmeraums und folglich entlang des gesamten in der Verpackung aufgenommenen Blasenkatheters vorgesehen, wodurch die Handhabbarkeit des erfindungsgemäßen Katheter-Sets in jeder Aus- bzw. Einführstellung des Blasenkatheters verbessert und erleichtert ist.

Vorteilhafterweise sind die Führungselemente äquidistant, bevorzugt mit fingerbreitem Abstand, angeordnet. Das den Blasenkatheter umgebende Verpackungsmaterial wird durch Zurückschieben entlang der axialen Erstreckung des Katheterschafts zusammengedrückt bzw. zusammengerafft und ermöglicht so das Vorwärtsschieben des Blasenkatheters. Die in regelmäßigen Abständen angeordneten Führungselemente erleichtern das Verschieben der Verpackung entlang des Katheterschafts.

In einer bevorzugten Ausführungsform der Erfindung sind die Führungselemente an der Oberseite und der Unterseite der Verpackung vorgesehen. Durch die beidseitige Anordnung von Führungselementen werden alle den Katheterschaft umgreifenden Finger eines Benutzers gegen Verrutschen gesichert und somit das Ein- bzw. Ausführen des Blasenkatheters aus der Verpackung weiter erleichtert.

Die Führungselemente sind bevorzugterweise als Raffungen, Riffelungen, Noppen und/oder Antirutschelemente ausgebildet. Eine derartige Ausbildung der Führungselemente aus dem folienartigen Material der Verpackung vereinfacht die Herstellung des erfindungsgemäßen Katheter-Sets. Es ist jedoch auch denkbar, die Führungselemente nachträglich, beispielsweise durch Aufkleben oder Beflocken, an der Verpackung anzubringen.

Es ist darüber hinaus vorteilhaft, dass der Blasenkatheter eine hydrophile Oberflächenbeschichtung und/oder eine Gleitmittelbeschichtung aufweist. Durch eine entsprechende Beschichtung werden die Gleiteigenschaften des Blasenkatheters verbessert, so dass dieser weitgehend schmerz- und irritationsfrei in den Körper des Patienten eingeführt werden kann.

In der Verpackung ist bevorzugt ein Reservoir mit einem Aktivierungsmittel und/oder einem Gleitmittel vorgesehen. Mit Hilfe des Aktivierungsmittels, beispielsweise einer Flüssigkeit wie Kochsalzlösung, wird die hydrophile Oberflächenbeschichtung des Blasenkatheters aktiviert, mit Hilfe des Gleitmittels, wie einem Gel, wird der Blasenkatheter benetzt und/oder aktiviert, wodurch die gewünschte Gleitfähigkeit des Blasenkatheters erreicht wird. Bei einem sehr gleitfähigen Blasenkatheter wirken sich die Führungselemente besonders vorteilhaft aus, da durch die friktionsarme Oberfläche des Blasenkatheters die Reibung zwischen dem Blasenkatheter und dem ihn umgebenden Katheteraufnahmeraum verringert ist und somit ein indirektes Verschieben des Blasenkatheters in oder aus der Verpackung heraus mit den erfindungsgemäßen Führungselementen erheblich erleichtert wird.

Besonders vorteilhaft ist, insbesondere bei einem Katheter-Set bei dem das Reservoir an einer Längsseite des Katheteraufnahmeraums am Katheteraufnahmeraum angeordnet ist, in der Verpackung eine Aktivierungs- und/oder Gleitmittelführung ausgebildet, die, nach einem Öffnen des Reservoirs, eine in Richtung der Katheterspitze gerichtete Benetzung der Oberfläche des Blasenkatheters mit Aktivierungsmittel und/oder Gleitmittel aus dem Reservoir unterstützt. Diese Aktivierungs- und/oder Gleitmittelführung ist in Richtung der Katheterspitze gerichtet und verbindet einen in der Verpackung ausgebildeten Reservoiraufnahmeraum, in dem das Reservoir angeordnet ist, mit dem Katheteraufnahmeraum. Im Falle der Verwendung einer wasserhaltigen Flüssigkeit als Aktivierungsmittel weist der Blasenkatheter bevorzugt eine hydrophile Oberflächenbeschichtung auf, um eine Aktivierung der Beschichtung des Blasenkatheters zu ermöglichen. Im Falle der Verwendung z.B. eines Gleitmittels erfolgt vor Applikation des Blasenkatheters eine Gleitmittelbenetzung der Oberfläche des Blasenkatheters. Durch eine entsprechende Beschichtung werden die Gleiteigenschaften des Blasenkatheters verbessert, so dass dieser weitgehend schmerz- und irritationsfrei in den Körper des Patienten eingeführt werden kann. Mit Hilfe des Aktivierungsmittels, beispielsweise Kochsalzlösung, wird eine hydrophile Oberflächenbeschichtung des Blasenkatheters aktiviert, mit Hilfe eines Gleitmittels, wie einem Gel, wird der Blasenkatheter benetzt und/oder aktiviert, wodurch die gewünschte Gleitfähigkeit des Blasenkatheters erreicht wird. Dadurch, dass mittels der Aktivierungs- und/oder Gleitmittelführung die Benetzung der Oberfläche des Blasenkatheters in Richtung der Katheterspitze gerichtet vorgenommen wird, kann mit weniger Aktivierungs- und/oder Gleitmittel ausgekommen werden. Das Aktivierungs- und/oder Gleitmittel fließt aus dem Reservoir direkt Richtung Katheterspitze, bzw. kann im Falle der Verwendung eines Gleitmittels problemlos zur Katheterspitze verschoben werden. Die vollständige Benetzung des vorderen Katheterteils, d.h. des katheterspitzennahen Teils des Blasenkatheters, wird vereinfacht und unterstützt. Da üblicherweise der Blasenkatheter nicht vollständig in die Harnröhre eingeschoben wird, sondern hauptsächlich der genannte vordere Katheterteil, reicht die Benetzung des vorderen Teils bereits zur Applikation des Blasenkatheters aus.

Bevorzugt ist die Aktivierungs- und/oder Gleitmittelführung als eine vom Reservoiraufnahmeraum schräg zum Katheteraufnahmeraum in Richtung der Katheterspitze geneigte, bevorzugt von einer Foliennaht ausgebildete, Flüssigkeitsführung ausgeführt. Derart lässt sich die Aktivierungs- und/oder Gleitmittelführung einfach durch Verbinden der die Verpackung ausbildenden Schichten des folienartigen Materials herstellen. Im. Falle der Verwendung von Kunststofffolie zur Herstellung der Verpackung kann diese Foliennaht einfach als Schweißnaht ausgeführt sein.

Wenn das Reservoir in einem Bereich in der Mitte der Längsausdehnung des Katheteraufnahmeraums in der Verpackung angeordnet ist, kann das Aktivierungsmittel und/oder das Gleitmittel leichter im Bereich der Oberfläche des Blasenkatheters verteilt werden, der in die Harnröhre einzuführen ist, als bei einer Anordnung z.B. am katheterspitzenabgewandten Ende des Katheters. Eine derartige Anordnung des Reservoirs ist insbesondere dann vorteilhaft, wenn der Katheteraufnahmeraum in Längsrichtung der Verpackung verläuft und die Verpackung im Wesentlichen rechteckförmig ausgebildet ist. Insbesondere wenn als Gleitmittel ein Gel verwendet wird, kann das Gel dann aus dem Reservoir einfach in den katheterspitzennahen Bereich des Katheteraufnahmeraums gedrückt werden. Da der Katheter mit seiner gesamten in die Harnröhre einzuführenden Länge durch diesen Bereich durchgeschoben wird, wird die Oberfläche des Katheters dadurch gleichmäßig mit dem Gel überzogen. Der Bereich in der Mitte der Längsausdehnung des Katheteraufnahmeraums kann z.B. im mittleren Drittel der Gesamtlänge der Verpackung liegen. Wenn am Reservoir oder in der Verpackung ein in den Katheteraufnahmeraum führender Kanal oder eine derartige Öffnung für das Aktivierungsmittel und/oder das Gleitmittel vorgesehen ist, sollten diese in dem Bereich in der Mitte der Längsausdehnung des Katheteraufnahmeraums angeordnet sein.

In einer bevorzugten Ausführungsform des erfindungsgemäßen Katheter-Sets sind in der Verpackung eine oder mehrere Grifföffnungen ausgebildet. Durch die Grifföffnungen wird die Handhabung des Katheter-Sets weiter erleichtert, beispielsweise kann das Katheter-Set während des Katheterisierungsvorgangs befestigt, beispielsweise an einem Ständer oder Haken aufgehängt werden.

Wenn in einem an die Austrittsstelle angrenzenden Bereich des Katheteraufnahmeraums eine Katheteraufnahmeraumverbreiterung vorgesehen ist, eignet sich der Katheteraufnahmeraum besonders gut für die Aufnahme eines Tiemannkatheter als Katheter des Katheter-Sets.
Ein Tiemannkatheter besitzt eine gebogene konisch zulaufende Katheterspitze. Durch seine Form eignet er sich besonders zur Katheterisierung der männlichen Harnblase, da die Spitze den Katheter besser durch die Harnröhre führt.

Es ist darüber hinaus denkbar, in den Katheteraufnahmeraum einen Katheter zur Entleerung und/oder Spülung eines beliebigen Körperhohlorgans, insbesondere zum Ableiten einer beliebigen Körperflüssigkeit, einzulegen. Ebenfalls lassen sich die Führungselemente der Verpackung zum verbesserten Auspacken eines Katheters nutzen und die erfindungsgemäße Ausbildung der Verpackung beschränkt sich nicht auf Blasenkatheter.

Weitere Vorteile und Merkmale der Erfindung ergeben sich aus der Beschreibung und den Figuren der Zeichnung. Ebenso können die vorstehend genannten und die noch weiter aufgeführten Merkmale erfindungsgemäß jeweils einzeln oder in beliebigen Kombinationen miteinander verwendet werden. Das erfindungsgemäße Katheter-Set ist in einem Ausführungsbeispiel in den Figuren gezeigt. Die in den Figuren gezeigten Merkmale sind rein schematisch und nicht maßstäblich zu verstehen. Es zeigt:
- Figur 1: eine Draufsicht eines erfindungsgemäßen Katheter-Sets;
- Figuren 2a und 2b: jeweils einen vergrößerten Ausschnitt eines erfindungsgemäßen Katheter-Sets;
- Figuren 3a und 3b: das als Verschlussklappe ausgebildete und an der Führungshülse angeformte Verschlusselement des erfindungsgemäßen Katheter-Sets aus Figur 1; und
- Figur 4: eine weitere Ausführungsform des Katheter-Sets.

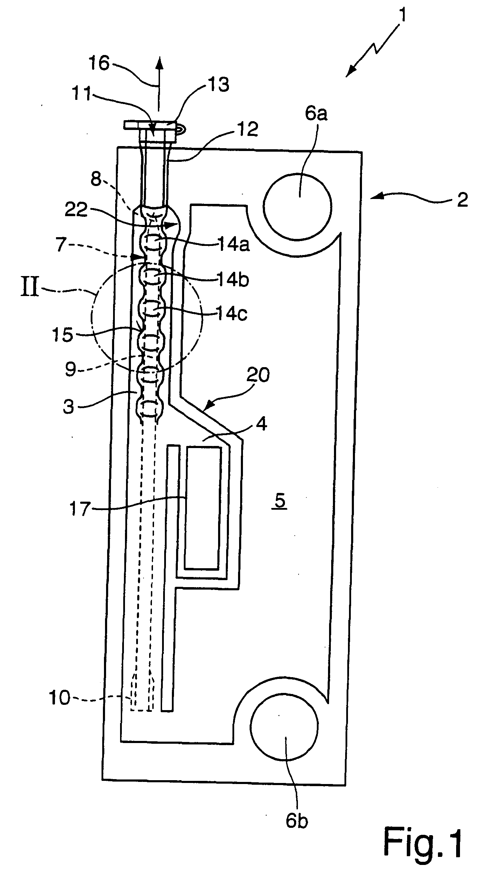

In Figur 1 ist in Draufsicht ein Katheter-Set 1 gezeigt, dessen Gestalt durch eine im Wesentlichen rechteckförmige Verpackung 2 bestimmt wird. Die Verpackung 2 besteht aus zwei miteinander verbundenen Lagen einer Kunststofffolie, in der ein länglicher, kanalartiger Katheteraufnahmeraum 3, ein Reservoiraufnahmeraum 4 und ein Urinaufnahmeraum 5 ausgebildet sind. Der Katheteraufnahmeraum 3 verläuft komplett oder teilweise in Längsrichtung der Verpackung 2. Der Reservoiraufnahmeraum 4 ist über den Katheteraufnahmeraum 3 mit dem Urinaufnahmeraum 5 verbunden und in einem Bereich in der Mitte der Längsausdehnung des Katheteraufnahmeraums 3, bzw. angrenzend an die Mitte des Katheteraufnahmeraums 3, angeordnet. An einem oder beiden schmalseitigen Enden der Verpackung 2 sind als kreisförmige Löcher ausgebildete Grifföffnungen 6a und 6b vorgesehen. In den Katheteraufnahmeraum 3 ist ein Blasenkatheter 7 eingelegt. Der Blasenkatheter 7 besteht aus einer Katheterspitze 8, einem langgestreckten Katheterschaft 9 und einem gegenüber dem Katheterschaft 9 verbreiterten Blockierelement 10. Der Katheteraufnahmeraum 3 wird am Rand der Verpackung 2 durch eine Austrittsstelle 11 begrenzt, auf die die Katheterspitze 8 des eingelegten Katheters 7 zeigt. An die Austrittsstelle 11 grenzt eine Führungshülse 12 an, bzw. die Austrittsstelle 11 wird von der Führungshülse 12 ausgebildet. In die Führungshülse 12 mündet der kanalartige Katheteraufnahmeraum 3. An der Führungshülse 12 ist ein als Verschlussklappe ausgebildetes Verschlusselement 13 vorgesehen, durch welches das durch den Katheteraufnahmeraum 3, den Reservoiraufnahmeraum 4 und den Urinaufnahmeraum 5 definierte Aufnahmevolumen der Verpackung 2 verschlossen ist. Der Blasenkatheter 7 kann in einer denkbaren Ausführungsform über die Verpackung 2 dauerhaft steril verpackt werden.
Der Reservoiraufnahmeraum 4 kann auch am unteren Ende des Katheteraufnahmeraums 3, d.h. im Bereich dessen austrittsstellenfernen Endes, angeordnet sein. Dann könnte der Katheteraufnahmeraum 3 über den Reservoiraufnahmeraum 4 mit dem Urinaufnahmeraum 5 verbunden sein.

Zum vereinfachten Ein- und Ausführen des Blasenkatheters 7 sind entlang der länglichen Erstreckung des Katheteraufnahmeraums 3 Führungselemente 14a, 14b, 14c, ... an einer Außenoberfläche 15 der Verpackung 2 vorgesehen. Durch die reihenförmig angeordneten Führungselemente 14a, 14b, 14c, ... , die als noppenartige Raffungen an der Außenoberfläche 15 ausgebildet sind, wird das Herausführen bzw. Herauslösen des Blasenkatheters 7 aus der Verpackung 2 in Ausführrichtung 16 erleichtert. Durch das Blockierelement 10 wird ein vollständiges Herausziehen des Blasenkatheters 7 aus der Verpackung 2 verhindert und der Katheteraufnahmeraum 3 kann beim Katheterisierungsvorgang nach außen abgedichtet werden. Im Reservoiraufnahmeraum 4 ist ein sachetartiges Reservoir 17 vorgesehen. In dem Reservoir 17 befindet sich ein Aktivierungsmittel zur Aktivierung einer hydrophilen Oberflächenbeschichtung des Blasenkatheters 7, insbesondere des Katheterschafts 9 und der Katheterspitze 8 oder ein Gel als ein Gleitmittel. Denkbar ist auch, dass der Reservoiraufnahmeraum direkt mit dem Aktivierungsmedium befüllt ist.

In der Verpackung ist eine Aktivierungs- und/oder Gleitmittelführung 20 ausgebildet, die den seitlich neben dem Katheteraufnahmeraum 3 im Bereich des Urinaufnahmeraums 5 angeordneten Reservoiraufnahmeraum 4 mit dem Katheteraufnahmeraum 3 verbindet. Die Aktivierungs- und/oder Gleitmittelführung 20 unterstützt eine in Richtung der Katheterspitze 8 gerichtete Benetzung der Katheteroberfläche mit Aktivierungsmittel und/oder Gleitmittel nach einem Öffnen des Reservoirs 17. Die Aktivierungs- und/oder Gleitmittelführung 20 ist dazu als eine schräg zum Katheteraufnahmeraum 3 in diese Richtung verlaufende Schweißnaht zwischen den die Verpackung 2 ausbildenden Kunststofffolien ausgeführt. Die Aktivierungs- und/oder Gleitmittelführung könnte z.B. auch durch einen entsprechend ausgerichteten, von zwei im Wesentlichen zueinander parallel verlaufenden Schweißnähten gebildeten Kanal als Flüssigkeitsführung ausgebildet sein. Der Winkel zwischen der Aktivierungs- und/oder Gleitmittelführung 20 und der Längsrichtung des Katheteraufnahmeraums 3 ist ein spitzer Winkel. Er beträgt bevorzugt ca. 30 bis 60 Grad.

In einem an die Austrittsstelle 11 angrenzenden Bereich des Katheteraufnahmeraums 3 ist eine Katheteraufnahmeraumverbreiterung 22 vorgesehen. Wenn anstelle des dargestellten Blasenkatheters 7 im Katheteraufnahmeraum 3 ein Tiemannkatheter, also ein Katheter mit gebogener Katheterspitze, vorgehalten werden soll, kann die gebogene Katheterspitze in der Katheteraufnahmeraumverbreiterung 22 ohne Verformung gelagert werden.

Die Handhabung des Katheter-Sets 1 ist folgendermaßen vorgesehen: Das Katheter-Set 1 wird mit geschlossenem Verschlusselement 13 in einer Sekundär-Verpackung oder sterilen Verpackung 2 geliefert. Das Reservoir 17 mit dem Aktivierungsmittel, einer sterilen Kochsalzlösung, wird durch Druck geöffnet, so dass das Aktivierungsmittel von dem Reservoiraufnahmeraum 4 in den Katheteraufnahmeraum 3 gelangen und den Blasenkatheter 7 benetzen kann. Durch das Aktivierungsmittel wird die hydrophile Oberflächenbeschichtung des Blasenkatheters 7 aktiviert und es ergeben sich verbesserte Gleiteigenschaften. Nach einer Wartezeit von ca. 30 Sekunden wird das Verschlusselement 13 geöffnet und der Blasenkatheter 7 kann durch die Führungshülse 12 aus der Verpackung 2 geschoben und über die Harnröhre in die Blase eines Benutzers eingeführt werden. Im Falle eines Gels als Gleitmittel im Reservoir 17 wird das Katheter-Set 1 mit offenem Verschlusselement 13 geliefert und das Gel wird aus dem Reservoir 17 in Richtung Katheterspitze 8 gedrückt und der Blasenkatheter 7 kann ohne Wartezeit sofort appliziert werden.
Der Urin aus der Blase läuft während des Katheterisierungsvorgangs direkt in die Verpackung 2, bzw. wird durch den Katheterschaft 9 und den Katheteraufnahmeraum 3 in den Urinaufnahmeraum 5 geleitet. Nach Abschluss der Katheterisierung wird der Blasenkatheter 7 aus der Blase und der Harnröhre wieder herausgezogen und zurück in den Katheteraufnahmeraum 3 geschoben und die Verpackung 2 wird mit Hilfe des Verschlusselements 13 wieder dicht verschlossen.

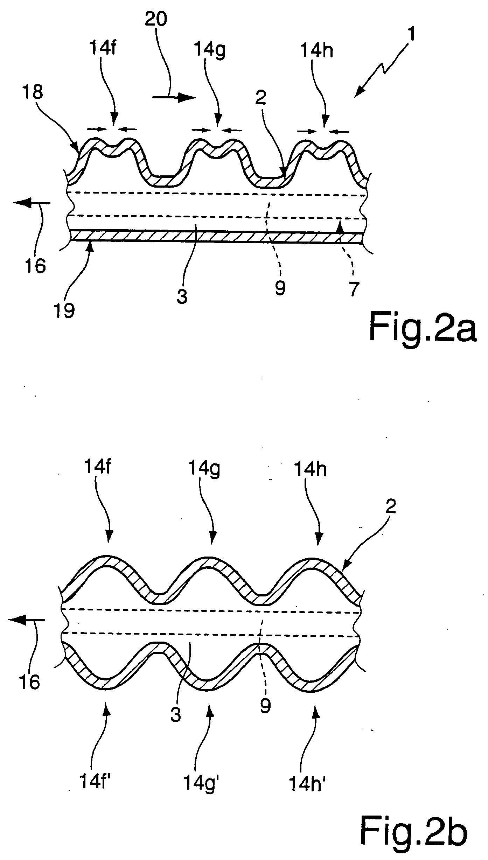

In Figur 2a ist ein Längsschnitt durch das in Figur 1 gezeigte Katheter-Set 1 im Ausschnitt II dargestellt. Der Längsschnitt verläuft entlang der axialen Erstreckung des Katheteraufnahmeraums 3 und des in ihn eingelegten Blasenkatheters 7. Die den Blasenkatheter 7 umhüllende bzw. umgebende Verpackung 2 weist an einer Oberseite 18 Führungselemente 14f, 14g und 14h auf. Durch die Aneinanderreihung der Führungselemente 14f, 14g und 14h erhält die Oberseite 18 eine wellige Kontur, wohingegen eine gegenüberliegende Unterseite 19 der Verpackung 2 glatt ausgeführt ist. Um den Blasenkatheter 7 in Ausführrichtung 16 aus dem Katheteraufnahmeraum 3 zu schieben, wird das flexible Material der Verpackung 2 entlang des Katheterschafts 9 in mehrfacher Wiederholung in einem Abschnitt zwischen beiden an der Verpackung 2 und dem Katheterschaft 9 angreifenden Händen gerafft bzw. verdichtet (mit Pfeilen angedeutet) und anschließend in einer der Ausführrichtung 16 entgegengesetzten Rückschieberichtung 20 gelängt bzw. zurückgeschoben. Das Verschieben der Verpackung 2 findet jeweils gegenüber einem ortsfesten Punkt statt, der durch eine der beiden Hände definiert wird. Die Konturen der Oberseite 18 und der Unterseite 19 der Verpackung 2 werden dabei verändert, wobei die Faltengebung an der Oberseite 18 durch die Führungselemente 14f, 14g und 14h vergleichbar einem Raffband vorgegeben ist. Dadurch werden allzu große Materialverdichtungen, welche sich nur schwer entlang des Katheterschafts 9 verschieben lassen, weitestgehend vermieden.

Der in Figur 2b gezeigte Längsschnitt unterscheidet sich von dem in Figur 2a gezeigten Schnitt darin, dass sowohl an der Oberseite 18 als auch an der Unterseite 19 der Verpackung 2 Führungselemente 14f, 14f, 14g, 14g',... vorgesehen sind. Die Führungselemente 14f, 14f', 14g, 14g',... bilden auf der Ober- und der Unterseite 18, 19 jeweils eine unebene, wellenartige Kontur aus. Beim Bewegen des Blasenkatheters 7 in Ausführrichtung 16 kann beidseits des Katheteraufnahmeraums 3, d.h. sowohl an der Oberseite 18 als auch an der Unterseite 19, der Katheterschaft 9 von einem Benutzer in abrutschsicherer Weise ergriffen und das Material der Verpackung 2 in kontrollierter Weise verschoben werden.

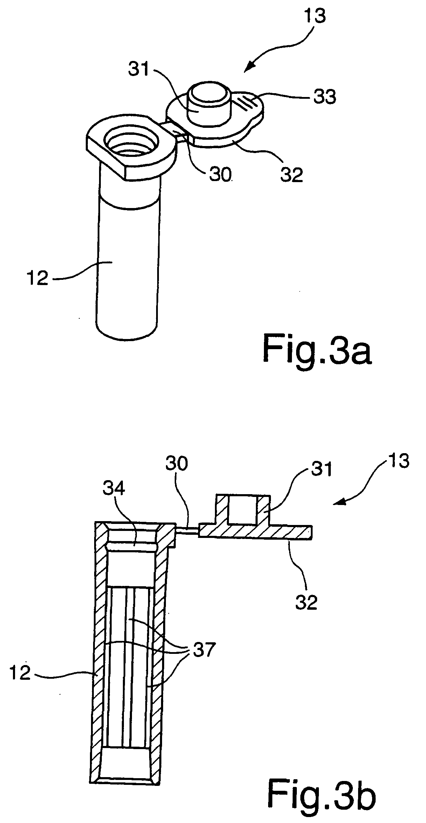

In den Figuren 3a und 3b ist das als Verschlussklappe ausgebildete und an der Führungshülse 12 angeformte Verschlusselement 13 des erfindungsgemäßen Katheter-Sets aus Figur 1 dargestellt. In Figur 3a ist eine dreidimensionale Darstellung gezeigt und in Figur 3b ist ein Längsschnitt gezeigt. Die Verschlussklappe und die Führungshülse 12 sind einstückig aus Kunststoff hergestellt. Um ein Auf- und Zuklappen der Verschlussklappe zu ermöglichen, ist diese über einen flexiblen Kunststoffstreifen 30 mit die Führungshülse 12 verbunden. Die Verschlussklappe wird von einem Kunststoffring 31 und einem über die radiale Außenabmessung der Führungshülse 12 hinausstehenden Deckelteil 32 ausgebildet. Dadurch, dass das Deckelteil 32 über die radiale Außenabmessung der Führungshülse 12 hinaussteht, lässt sich die Verschlussklappe leicht öffnen. Der Deckel 32 weist auf seiner im verschlossenen Zustand der Führungshülse 12 zugewandten Unterseite eine Riffelung 33 zur Verbesserung seiner Griffigkeit auf. Der Kunststoffring 31 bildet einen Pfropfen zum Verschließen der Austrittsstelle 11 aus. Da der Kunststoffring 31 beim Verschließen der Führungshülse 12 radial leicht zusammengepresst wird, ergibt sich eine sehr gute Dichtigkeit gegen Ausfließen von Harn. Eine umlaufende Dichtlippe 34 ist an der Innenoberfläche der Führungshülse 12 angeordnet. Da diese Dichtlippe 34 den Innendurchmesser der Führungshülse 12 im Bereich, in dem der Kunststoffring zum Verschließen in die Führungshülse 12 eingesteckt wird, verengt, erleichtert die Dichtlippe 34 das radiale Zusammenpressen des Kunststoffrings 31 beim Verschließen der Führungshülse 12. Eine entsprechende Dichtlippe kann auch radial umlaufend am Pfropfen vorgesehen sein.
In Figur 3b sind an der Innenoberfläche der Führungshülse 12 in Katheteraustrittsrichtung verlaufende Führungsrippen 37 zur zentrierten Führung des Blasenkatheters bei dessen Applikation zu erkennen.

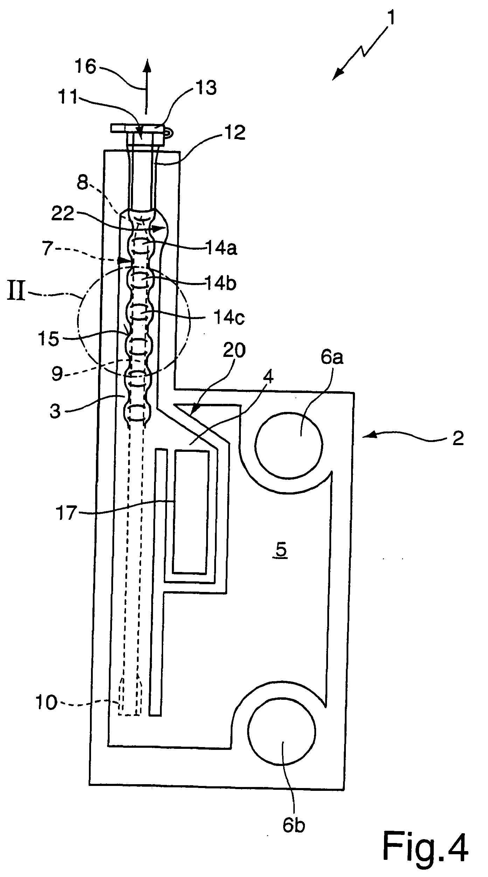

Figur 4 zeigt das erfindungsgemäße Katheter-Set mit einer veränderten äußeren Verpackungsform. Der Urinaufnahmeraum 5 erstreckt sich nicht über die gesamte Länge des Katheteraufnahmeraums und bietet somit gegenüber der Ausführungsform aus Fig. 1 veränderte Applikationsvorteile.

Vorgeschlagen wird ein Katheter-Set 1, umfassend eine aus einem folienartigen Material hergestellte Verpackung 2 und einen Blasenkatheter 7 mit einem flexiblen Katheterschaft 9 und einer Katheterspitze 8, wobei die Verpackung 2 einen im Wesentlichen röhrenförmigen Katheteraufnahmeraum 3 aufweist, in den der Blasenkatheter 7 eingelegt ist, und wobei an dem der Katheterspitze 8 zugeordneten Ende des Katheteraufnahmeraums 3 eine Austrittsstelle 11 für den Blasenkatheter 7 vorgesehen ist. Der Katheteraufnahmeraum 3 ist als Teil eines Urinaufnahmeraums 5 ausgeführt und an der Austrittsstelle 11 ist ein wiederverschließbares Verschlusselement 13 vorgesehen.

Die Erfindung beschränkt sich nicht auf die vorstehend angegebenen Ausführungsbeispiele. Vielmehr ist eine Anzahl von Varianten denkbar, welche auch bei grundsätzlich anders gearteter Ausführung von den Merkmalen der Erfindung Gebrauch machen.

## Patentansprüche

1. Katheter-Set (1) umfassend
eine aus einem folienartigen Material hergestellte Verpackung (2) und einen Blasenkatheter (7) mit einem flexiblen Katheterschaft (9) und einer Katheterspitze (8),
wobei die Verpackung (2) einen im Wesentlichen röhrenförmigen Katheteraufnahmeraum (3) aufweist, in den der Blasenkatheter (7) eingelegt ist, und
wobei an dem der Katheterspitze (8) zugeordneten Ende des Katheteraufnahmeraums (3) eine Austrittsstelle (11) für den Blasenkatheter (7) vorgesehen ist und der Katheteraufnahmeraum (3) als Teil eines Urinaufnahmeraums (5) ausgeführt ist und an der Austrittsstelle (11) ein wiederverschließbares Verschlusselement (13) vorgesehen ist, und der Katheteraufnahmeraum (3) eine an die Austrittsstelle (11) angrenzende Führungshülse (12) aufweist, wobei das Verschlusselement (13) als Verschlussklappe an der Führungshülse (12) ausgebildet ist, **dadurch gekennzeichnet, dass** die Führungshülse (12) an ihrer Innenoberfläche in Katheterausführrichtung (16) verlaufende Führungsrippen (37) aufweist.

2. Katheter-Set nach Anspruch 1, **dadurch gekennzeichnet, dass** die Verschlussklappe und die Führungshülse (12) einstückig aus Kunststoff hergestellt sind, wobei die Verschlussklappe und die Führungshülse (12) über mindestens einen flexiblen Kunststoffstreifen (30) miteinander verbunden sind.

3. Katheter-Set nach einem der vorhergehenden Ansprüche, **dadurch gekennzeichnet, dass** die Führungshülse (12) und/oder das Verschlusselement (13) eine umlaufende Dichtlippe (34) aufweisen.

4. Katheter-Set nach einem der vorhergehenden Ansprüche, **dadurch gekennzeichnet, dass** in zumindest einem Längenabschnitt des Katheteraufnahmeraums (3) Führungselemente (14a, 14b, ...) an der Außenoberfläche (15) der Verpackung (2) vorgesehen sind und dass die Führungselemente (14a, 14b, ...) in Längsrichtung des Katheteraufnahmeraums (3) der Verpackung (2) dem Verlauf des Katheteraufnahmeraums (3) zumindest teilweise folgen.

5. Katheter-Set nach Anspruch 4, **dadurch gekennzeichnet, dass** die Führungselemente (14a, 14b, ...) im Bereich der Austrittsstelle (11) vorgesehen sind.

6. Katheter-Set nach Anspruch 4, **dadurch gekennzeichnet, dass** sich die Führungselemente (14a, 14b,...) über die gesamte Länge des Katheteraufnahmeraums (3) erstrecken.

7. Katheter-Set nach einem der Ansprüche 4 bis 6, **dadurch gekennzeichnet, dass** die Führungselemente (14a, 14b, ...) äquidistant angeordnet sind.

8. Katheter-Set nach einem der Ansprüche 4 bis 7, **dadurch gekennzeichnet, dass** die Führungselemente (14a, 14b,...) an der Oberseite (18) und der Unterseite (19) der Verpackung (2) vorgesehen sind.

9. Katheter-Set nach einem der Ansprüche 4 bis 8, **dadurch gekennzeichnet, dass** die Führungselemente (14a, 14b, ...) als Raffungen, Riffelungen, Noppen und/oder Antirutschelemente ausgebildet sind.

10. Katheter-Set nach einem der vorhergehenden Ansprüche, **dadurch gekennzeichnet, dass** der Blasenkatheter (7) eine hydrophile Oberflächenbeschichtung und/oder eine Gleitmittelbeschichtung aufweist.

11. Katheter-Set nach einem der vorhergehenden Ansprüche, **dadurch gekennzeichnet, dass** in der Verpackung (2) ein Reservoir (17) mit einem Aktivierungsmittel und/oder einem Gleitmittel vorgesehen ist oder das Aktivierungsmedium direkt im Reservoiraufnahmeraum abgefüllt ist.

12. Katheter-Set nach Anspruch 11, **dadurch gekennzeichnet, dass** das Reservoir (17) an einer Längsseite des Katheteraufnahmeraums (3) am Katheteraufnahmeraum (3) angeordnet ist, wobei in der Verpackung (2) eine in Richtung der Katheterspitze (8) gerichtete Aktivierungs- und/oder Gleitmittelführung (20), zur Unterstützung einer in Katheterspitzenrichtung gerichteten Benetzung der Oberfläche des Blasenkatheters (7) mit dem Aktivierungsmittel und/oder Gleitmittel aus dem Reservoir (17), ausgebildet ist, die einen in der Verpackung (2) ausgebildeten Reservoiraufnahmeraum (4), in dem das Reservoir (17) angeordnet ist, mit dem Katheteraufnahmeraum (3) verbindet.

13. Katheter-Set nach Anspruch 12, **dadurch gekennzeichnet, dass** die Aktivierungs- und/oder Gleitmittelführung (20) als eine schräg zum Katheteraufnahmeraum (3) in Richtung der Katheterspitze (8) geneigte, bevorzugt von einer Foliennaht ausgebildete, Flüssigkeitsführung ausgeführt ist.

14. Katheter-Set nach Anspruch 11, **dadurch gekennzeichnet, dass** das Reservoir (17) in einem Bereich in der Mitte der Längsausdehnung des Katheteraufnahmeraums (3) in der Verpackung (2) angeordnet ist, bevorzugt wobei der Katheteraufnahmeraum (3) in Längsrichtung der Verpackung (2) verläuft und die Verpackung im Wesentlichen (2) rechteckförmig ausgebildet ist.

15. Katheter-Set nach einem der vorhergehenden Ansprüche, **dadurch gekennzeichnet, dass** in der Verpackung (2) eine oder mehrere Grifföffnungen (6a, 6b) ausgebildet sind.

16. Katheter-Set nach einem der vorhergehenden Ansprüche, **dadurch gekennzeichnet, dass** in einem an die Austrittsstelle (11) angrenzenden Bereich des Katheteraufnahmeraums (3) eine Katheteraufnahmeraumverbreiterung (22) vorgesehen ist.

## Claims

1. A catheter set (1) comprising
a package (2) produced from a foillike material and a urinal catheter (7) having a flexible catheter shaft (9) and a
catheter tip (8),
wherein the package (2) comprises a substantially tubular catheter storage space (3) having the urinal catheter (7) inserted therein, and
wherein an exit location (11) for the urinal catheter (7) is provided at the end of the catheter storage space (3) associated with the catheter tip (8), and the catheter storage space (3) is implemented as part of a urine storage space (5), and a reclosable closure element (13) is provided at the exit location (11), and the catheter storage space (3) is provided with a guide sleeve (12) adjoining the exit location (11), the closure element (13) being implemented as a closure flap on said guide sleeve (12), **characterized in that** the inner surface of the guide sleeve (12) is provided with guide ribs (37) extending in the catheter removal direction (16).

2. A catheter set according to claim 1, **characterized in that** the closure flap and the guide sleeve (12) are produced from plastic material in one piece, said closure flap and said guide sleeve (12) being connected to one another by at least one strip of flexible plastic material (30).

3. A catheter set according to one of the preceding claims, **characterized in that** the guide sleeve (12) and/or the closure element (13) are provided with a circumferentially extending sealing lip (34).

4. A catheter set according to one of the preceding claims, **characterized in that** guide elements (14a, 14b, ...) are provided on the outer surface (15) of the package (2) in at least one longitudinal section of the catheter storage space (3), and that the guide elements (14a, 14b, ...) follow the profile of the catheter storage space (3) at least partially in the longitudinal direction of the catheter storage space (3) of the package (2).

5. A catheter set according to claim 4, **characterized in that** the guide elements (14a, 14b, ...) are provided in the area of the exit location (11).

6. A catheter set according to claim 4, **characterized in that** the guide elements (14a, 14b, ...) extend over the entire length of the catheter storage space (3).

7. A catheter set according to one of the claims 4 to 6, **characterized in that** the guide elements (14a, 14b, ...) are arranged in an equidistant fashion.

8. A catheter set according to one of the claims 4 to 7, **characterized in that** the guide elements (14a, 14b, ...) are provided on the upper side (18) and on the lower side (19) of the package (2).

9. A catheter set according to one of the claims 4 to 8, **characterized in that** the guide elements (14a, 14b, ...) are implemented as gathers, corrugations, knobs and/or anti-skid elements.

10. A catheter set according to one of the preceding claims, **characterized in that** the urinal catheter (7) is provided with a hydrophilic surface coating and/or a lubricant coating.

11. A catheter set according to one of the preceding claims, **characterized in that** the package (2) has provided therein a reservoir (17) containing an activating agent and/or a lubricant or that the activating agent is directly filled into the reservoir storage space.

12. A catheter set according to claim 11, **characterized in that** the reservoir (17) is arranged on the catheter storage space (3) at a longitudinal side of said catheter storage space (3), wherein the package (2) has formed therein an activating agent and/or lubricant guide (20) directed towards the catheter tip (8) and used for supporting a catheter wetting process in which the surface of the urinal catheter (7) is wetted in the direction of the catheter tip with the activating agent and/or lubricant from the reservoir (17), said activating agent and/or lubricant guide (20) interconnecting a reservoir storage space (4), which is formed in the package (2) and in which the reservoir (17) is arranged, and the catheter storage space (3).

13. A catheter set according to claim 12, **characterized in that** the activating agent and/or lubricant guide (20) is configured as a fluid guide which is inclined at an oblique angle relative to the catheter storage space (3) in the direction of the catheter tip (8) and which is preferably defined by a foil seam.

14. A catheter set according to claim 11, **characterized in that** the reservoir (17) is arranged in the package (2) in an area in the middle of the length of the catheter storage space (3), preferably the catheter storage space (3) extending in the longitudinal direction of the package (2), and the package (2) being substantially rectangular.

15. A catheter set according to one of the preceding claims, **characterized in that** the package (2) is provided with one or more handle openings (6a, 6b).

16. A catheter set according to one of the preceding claims, **characterized in that** a catheter storage space enlargement (22) is provided in an area of the catheter storage space (3) adjoining the exit location (11).

## Revendications

1. Set de cathéter (1) comprenant
un emballage (2) fabriqué en un matériau en forme de feuille, et un cathéter à ballonnet (7) avec une tige de cathéter (9) flexible et une pointe de cathéter (8), set dans lequel l'emballage (2) présente un logement d'accueil de cathéter (3) sensiblement de forme tubulaire et dans lequel est placé le cathéter à ballonnet (7), et
dans lequel il est prévu, à l'extrémité du logement d'accueil de cathéter (3), qui est associée à la pointe de cathéter (8), une zone de sortie (11) pour le cathéter à ballonnet (7), et le logement d'accueil de cathéter (3) est réalisé en tant que partie d'une chambre de collecte d'urine (5), et au niveau de la zone de sortie (11) il est prévu un élément de fermeture (13) refermable, et le logement d'accueil de cathéter (3) comprend un fourreau de guidage (12) adjacent à la zone de sortie (11), l'élément de fermeture (13) étant réalisé sous forme de clapet de fermeture sur le fourreau de guidage (12),
**caractérisé en ce que** le fourreau de guidage (12) présente sur sa surface intérieure, des nervures de guidage (37) s'étendant dans la direction d'extraction de cathéter (16).

2. Set de cathéter selon la revendication 1, **caractérisé en ce que** le clapet de fermeture et le fourreau de guidage (12) sont fabriqués d'un seul tenant en matière plastique, le clapet de fermeture et le fourreau de guidage (12) étant reliés mutuellement par l'intermédiaire d'au moins une bande de matière plastique (30) flexible.

3. Set de cathéter selon l'une des revendications précédentes, **caractérisé en ce que** le fourreau de guidage (12) et/ou l'élément de fermeture (13) présentent une lèvre d'étanchéité (34) périphérique.

4. Set de cathéter selon l'une des revendications précédentes, **caractérisé en ce que** dans au moins un tronçon de longueur du logement d'accueil de cathéter (3), sont prévus des éléments de guidage (14a, 14b, ...) sur la surface extérieure (15) de l'emballage (2), et **en ce que** les éléments de guidage (14a, 14b, ...) suivent, dans la direction longitudinale du logement d'accueil de cathéter (3) de l'emballage (2), au moins partiellement le tracé du logement d'accueil de cathéter (3).

5. Set de cathéter selon la revendication 4, **caractérisé en ce que** les éléments de guidage (14a, 14b, ...) sont prévus dans la région de la zone de sortie (11).

6. Set de cathéter selon la revendication 4, **caractérisé en ce que** les éléments de guidage (14a, 14b, ...) s'étendent sur la totalité de la longueur du logement d'accueil de cathéter (3).

7. Set de cathéter selon l'une des revendications 4 à 6, **caractérisé en ce que** les éléments de guidage (14a, 14b, ...) sont agencés de manière équidistante les uns des autres.

8. Set de cathéter selon l'une des revendications 4 à 7, **caractérisé en ce que** les éléments de guidage (14a, 14b, ...) sont prévus sur le côté supérieur (18) et le côté inférieur (19) de l'emballage (2).

9. Set de cathéter selon l'une des revendications 4 à 8, **caractérisé en ce que** les éléments de guidage (14a, 14b, ...) sont réalisés sous forme de fronces, de cannelures, de picots et/ou d'éléments antidérapants.

10. Set de cathéter selon l'une des revendications précédentes, **caractérisé en ce que** le cathéter à ballonnet (7) présente un revêtement de surface hydrophile et/ou un revêtement lubrifiant ou antifriction.

11. Set de cathéter selon l'une des revendications précédentes, **caractérisé en ce que** dans l'emballage (2) est prévu un réservoir (17) avec un activateur et/ou un agent lubrifiant ou antifriction, ou bien l'agent activant est rempli directement dans le logement de réservoir.

12. Set de cathéter selon la revendication 11, **caractérisé en ce que** le réservoir (17) est agencé au niveau du logement d'accueil de cathéter (3), sur un côté longitudinal du logement d'accueil de cathéter (3), et dans l'emballage (2) est formé un guide d'activateur et/ou d'agent lubrifiant (20), qui est dirigé en direction de la pointe de cathéter (8), pour contribuer à un mouillage, dirigé vers la pointe de cathéter, de la surface du cathéter à ballonnet (7) avec l'activateur et/ou l'agent lubrifiant en provenance du réservoir (17), et qui relie un logement d'accueil de réservoir (4) formé dans l'emballage (2) et dans lequel est agencé le réservoir (17), au logement d'accueil de cathéter (3).

13. Set de cathéter selon la revendication 12, **caractérisé en ce que** le guide d'activateur et/ou d'agent lubrifiant (20) est réalisé en tant que guide de liquide, de préférence formé par un joint soudé de feuille, qui s'étend de manière oblique par rapport au logement d'accueil de cathéter (3) en direction de la pointe de cathéter (8).

14. Set de cathéter selon la revendication 11, **caractérisé en ce que** le réservoir (17) est agencé dans une zone située au milieu de l'étendue en longueur du logement d'accueil de cathéter (3) dans l'emballage (2), le logement d'accueil de cathéter (3) s'étendant de préférence dans la direction longitudinale de l'emballage (2) et l'emballage (2) étant sensiblement de configuration rectangulaire.

15. Set de cathéter selon l'une des revendications précédentes, **caractérisé en ce que** dans l'emballage (2) sont formées une ou plusieurs ouvertures de préhension (6a, 6b).

16. Set de cathéter selon l'une des revendications précédentes, **caractérisé en ce que** dans une région du logement d'accueil de cathéter (3), qui est adjacente à la zone de sortie (11), il est prévu un élargissement de logement d'accueil de cathéter (22).
